# EUROPEAN PATENT SPECIFICATION

(11) **EP 2 624 909 B1**
(45) Date of publication and mention of the grant of the patent: **15.11.2017**
(21) Application number: 11773558.9
(22) Date of filing: 29.09.2011
(51) Int. Cl.: A61N 1/39, A61N 1/37

(54) **MODE KNOB WITH TIME CRITICALITY ORDERING OF MODES**
BETRIEBSMODUSKNOPF MIT ZEITKRITISCHEN MODUSBEFEHLEN
BOUTON DE MODE À CLASSEMENT DE MODES PAR CRITICITÉ TEMPORELLE

(30) Priority: 11.11.2010 US 412564 P; 08.10.2010 US 391426 P
(43) Date of publication of application: 14.08.2013
(73) Proprietor: Koninklijke Philips N.V., 5656 AE Eindhoven (NL)
(72) Inventor: HIGLEY, Virginia, 5656 AE Eindhoven (NL)
(74) Representative: Steffen, Thomas
(86) International application number: PCT/IB2011/054286
(87) International publication number: WO 2012/046168

(56) References cited:
- US-A- 3 442 269
- US-A- 3 865 101
- US-A1- 2004 111 122
- US-A1- 2006 282 124

## Description

### Background of the Invention

The present invention relates to defibrillators for cardiac resuscitation. The invention relates in particular to a novel arrangement of selections on a mode control user interface in a defibrillator.

Cardiac arrest is a life-threatening medical condition in which the patient's heart fails to provide blood flow to support life. A defibrillator can be used to deliver defibrillating shocks to a patient suffering from cardiac arrest or other arrhythmia. The defibrillator resolves this condition by delivering a high-voltage impulse to the heart in order to restore normal rhythm and contractile function in patients who are experiencing cardiac arrhythmia. VF (ventricular fibrillation) or VT (ventricular tachycardia) is immediately life-threatening arrhythmias that are typically nonperfusing, i.e. not accompanied by spontaneous circulation. AF (atrial fibrillation) is an arrhythmia which is potentially life-threatening, but is accompanied by spontaneous circulation. Each type of arrhythmia may be best treated with a different type of defibrillation shock. For example, AF is treated with a synchronized shock (cardio-version). VF and unstable VT are treated with an unsynchronized shock.

External defibrillators typically act through hand-held electrode paddles or adhesivelyapplied electrode pads applied across the chest of the patient. The electrodes are used to apply the defibrillating shock and can also be used to acquire an ECG signal from the patient's heart. Paddle electrodes typically have a shock delivery button or buttons on the paddles. Defibrillators which employ adhesive electrodes typically have the shock delivery button on the front control panel of the defibrillator. Many advanced defibrillators accept both paddle and adhesive electrodes: therefore a user may have one or more choices of shock delivery control depending on the electrode type employed.

Defibrillators suitable for use in advanced life support or hospital environments often have a number of modes of operation. Each mode treats a different kind of cardiac event. Each event in turn has a different level of urgency in providing treatment.

For example, defibrillation of non-perfusing arrhythmias such as VF or VT must be delivered very soon after the onset of cardiac arrest in order to be effective. It is estimated that the chance of survival falls by 10% for every minute of delay to defibrillation beyond four minutes after cardiac arrest. Hence, it is vital that once a defibrillator is brought to the patient, the rescuer deploy and use it quickly.

The automated external defibrillator (AED) mode is especially effective for the treatment of non-perfusing arrhythmias. In the AED mode, the defibrillator automatically analyzes the electrocardiogram (ECG) rhythm of an unconscious patient to quickly ascertain whether defibrillation is necessary. The defibrillator analyzes the ECG signal for signs of arrhythmia. If VF is detected, the defibrillator signals the rescuer that a shock is advised and charges its electrotherapy circuit to a therapeutic energy. The rescuer must only press a shock button on the defibrillator to deliver a defibrillation pulse to resuscitate the patient.

In the manual mode of operation, the defibrillator allows the user to select a desired amount of defibrillation energy and apply that energy directly to the patient without previous automatic diagnosis. Generally, only the minimum amount of energy necessary to convert the particular arrhythmia is desired, because too much energy can potentially injure the heart or create other complications. The user typically selects the energy level by turning a knob on the defibrillator control panel to one of several so-labeled positions. The manual mode can be used to treat arrhythmias that don't require a synchronized shock, but requires a more highly trained user to diagnose the underlying rhythm and select the proper therapeutic energy.

Some advanced defibrillators incorporate a synchronized cardio-version defibrillation mode. Synchronized cardio-version treats arrhythmias in an underlying, typically perfusing, heart rhythm such as AF, stable VT, atrial flutter, and supraventricular tachycardias (SVT). Such arrhythmias are not normally immediately life-threatening. In the synchronized cardio-version mode, the defibrillator monitors the ECG and delivers the shock at a certain point in the heart rhythm in order to reduce the risk of initiating fibrillation.

Advanced defibrillators may also incorporate a pacing mode of operation. Pacing comprises a lower energy series of shocks designed to control the rate of the pumping heart. A pacing pulse corresponds to and triggers each heartbeat. Pacing is generally applied to stabilize a patient's perfusing rhythm.

In the monitoring mode of operation, defibrillation and pacing is inactivated altogether. A user selects monitoring mode in a defibrillator for use on a stable patient. The defibrillator typically displays the patient's ECG and analyzes the ongoing ECG for deterioration or instability. If an instability is detected, the defibrillator issues a visual or aural alert.

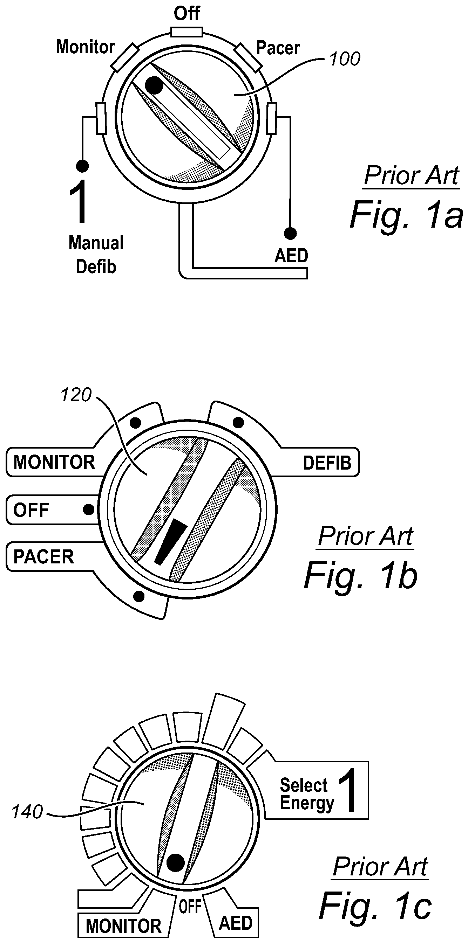

Selection of monitoring mode is often by the same knob used to select manual mode defibrillation energies, AED mode, synchronized cardio-version mode and/or pacing mode of operation. Examples of selector knobs in prior art advanced defibrillators are shown in FIGURES 1a - 1c. In the prior art devices the monitor mode position is placed adjacent to the "Off" position.

The location of the monitor mode on the selector knob presents several problems. First, in the confusion and extreme urgency of emergencies involving cardiac arrest, a rescuer may mistakenly turn the selector knob to "monitor" instead of to the "AED" mode or manual defibrillation mode. The probability of mistake is heightened if the rescuer is unfamiliar with the defibrillator's setup and operation. Unnecessary delay to defibrillation occurs if the mistake is not discovered until a shock is unsuccessfully attempted. Delay to defibrillation may also occur if the rescuer must navigate through a number of non-defibrillation modes of operation before arriving at the proper defibrillation mode. What is needed is a mode selection knob which reduces confusion and delay.

One defibrillator is described in US 3,442,269. Here a defibrillator having a control circuit incorporated in a defibrillator-cardioscope unit is disclosed. The defibrillator includes a selector switch having a plurality of control positions for variously interrelating the operation of the defibrillator and the cardioscope.

### Summary of the Invention

The present invention arose from the discovery that the prior art arrangement of modes of operation on an advanced defibrillator mode selection knob incurs delay to defibrillation and increases the chance for making an error in selecting the mode during a sudden cardiac arrest event. As shown in FIGURES 1a - 1c, the mode selection knobs on prior art defibrillators typically place the monitor mode of operation adjacent to the "Off" position. While this configuration may make the selection of monitoring more convenient, and might avoid problems with momentarily energizing a defibrillation-related circuit, it creates other and more severe problems for urgent care of sudden cardiac arrests.

In accordance with the principles of the present invention, a defibrillator is described having a number of different operating modes. The defibrillator has an improved mode selection knob for selecting the operating mode. The improved mode selection knob is disposed such that the most time-critical modes of operation are placed nearer the "Off" position, and the less time-critical modes of operation are placed in positions away from the "Off" position.

In accordance with another aspect of the invention, a defibrillator is described having a number of different operating modes. The defibrillator has an improved mode selection knob for selecting the operating mode. The improved mode selection knob is disposed such that positions for an AED mode of operation and a manual defibrillation
mode of operation "straddle" the "Off" position. This invention enables the quick initiation of a defibrillation mode for a cardiac rescue without requiring the operator to cycle through other non-rescue modes of operation.

In accordance with another aspect of the invention, a defibrillator is described having a number of different operating modes. The defibrillator has an improved mode selection knob for selecting the operating mode. The improved mode selection knob is disposed such that positions for the various modes of operation are ranked in order of time-criticality of rescue. The positions are arranged about the mode selection knob in order of time-criticality, such that the most time-critical
function is located closest to the default "Off" position, and the least time-critical function is located furthest from the default "Off" position.

### Brief Description of the Several Views of Drawings

In the drawings:
FIGURES 1a, 1b, and 1c illustrate several prior art defibrillator mode selection selector switches.
FIGURE 2 illustrates a defibrillator mode selector switch in which the mode of operation positions are ordered by time-criticality. The AED mode, being most time-critical, is arranged next to the "Off" position, whereas the Monitor mode, being the least time-critical, is arranged furthest from the "Off" position.
FIGURE 3 illustrates a defibrillator mode selector switch in which a manual defibrillation mode position and an AED mode position straddle the "Off" position.

### Detailed Description of the Invention

The example of FIGURE 1a, a mode selection knob for a defibrillator manufactured by Mindray Corporation, illustrates the problem solved by the present invention. Mode selection knob 100 is arranged such that in the default "Off" position, the user must cycle through either the "Monitor" position or the "Pacer" position to get to a defibrillation mode of operation. Such an arrangement at least requires additional time to cycle the knob to the more time-critical modes of defibrillation, and also poses the risk of the user not turning the mode selection knob far enough. The mistake could inhibit or delay necessary defibrillation treatment.

FIGURE 1b, illustrating a mode selection knob for a defibrillator manufactured by Zoll Corporation, also illustrates a similar problem recognized and solved by the present invention. Mode selection knob 120 is arranged such that in the default "Off" position, the user must cycle through the "Monitor" position to get to a defibrillation mode of operation. The "Pacer" position, which provides no emergency electrotherapy, is located adjacent the "Off" position as well. Such an arrangement also requires additional time to cycle the knob to the more time-critical mode of defibrillation and poses an increased risk of the user mistakenly selecting a non-defibrillation mode exactly when it is needed most.

FIGURE 1c illustrates a somewhat better, but still non-ideal, arrangement of positions on a defibrillator mode selection switch. In this defibrillator, manufactured by Philips Electronics North America, the mode selection knob arranges the time-critical AED mode adjacent to the "off" position. However, on the other side of the "off" position is located the "monitor" position. Thus, this defibrillator also carries some risk of the user mistakenly selecting a non-defibrillation mode exactly when it is most needed.

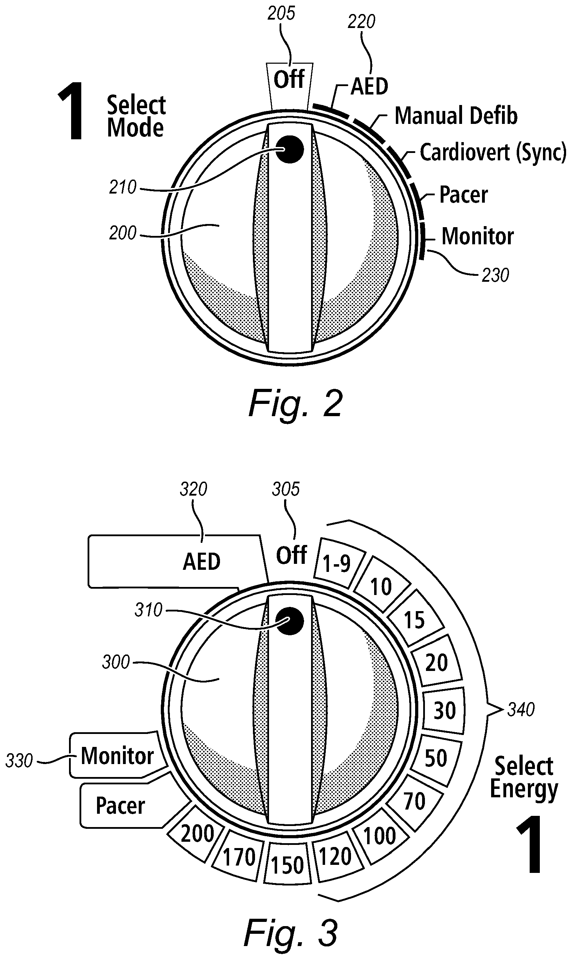

FIGURE 2 illustrates one embodiment of the present invention. Here shown is a rotary mode selector switch 200 for a multi-function defibrillator. Switch 200 is preferably shaped for easy grasping and rotation, and preferably comprises a selector position indicator 210 which shows the current position of the switch. The operating mode at each switch position is preferably delimited by combinations of text, markings, and symbols applied to the panel adjacent the switch in any manner similar to those shown in FIGURES 2 and 3.

The rotatable mode selector switch 200 shown in FIGURE 2 has at least three positions. An Off position 205 de-energizes or inactivates the device. Optionally, Off position 205 may place the device into a standby operating mode. A first position which energizes a mode for delivering electrotherapy, such as the AED position 220, is placed adjacent the Off position. A second position which energizes a non-electrotherapy mode of operation, such as the Monitor position 230, is placed at a position that is not adjacent to the Off position. Preferably, the Monitor position 230 is positioned such that a user must turn the mode selector switch 200 through the AED position 220 in order to reach the Monitor position 230. In the preferred embodiment, mode selector switch 200 is configured such that it can only be turned in the clockwise direction from the OFF position 205.

The mode selector switch 200 of FIGURE 2 may optionally be configured such that the user may turn the switch from Off to Monitor in either of the clockwise or counterclockwise direction. In this embodiment, the Monitor position 230 is also not adjacent to the Off position 205, because it is substantially more distant than the AED position 220 from the Off position 205. For example, FIGURE 2 illustrates that AED position 220 lies approximately 18 degrees clockwise from the Off position 205, whereas Monitor position 230 lies 270 degrees counterclockwise from the Off position 205.

FIGURE 2 also shows operating modes in addition to the AED mode 220 and Monitor mode 230. Shown are operating modes described previously; Manual Defib mode, Cardiovert (Sync) mode for synchronized cardio-version, and Pacer mode. Preferably, each of these modes is arranged clockwise around the mode selector switch 200 in order of decreasing time-criticality of the underlying therapy for the indicated cardiac arrhythmia. It is noted in this embodiment that the Manual Defib mode for manual defibrillation includes no specific selectable energy levels. Energy selection would thus be accomplished on a separate user control, not illustrated here.

Turning now to FIGURE 3, an alternate embodiment of the model selector switch 300 is illustrated. Similar to the embodiment of FIGURE 2, mode selector switch 300 comprises the three positions of Off position 305, AED position 320, and Monitor position 330. Device functionality associated with these positions is similar to that described previously. Mode selector switch 300 further comprises a number of sub-positions 340 representing selectable energy levels for manual defibrillation. Typically, energy positions in manual defibrillation range from approximately 1 Joule to 200 or more Joules, depending on the particular arrhythmia to be treated and the physical characteristics of the patient.

In the FIGURE 3 embodiment, the AED position 320 on the selector switch 300 is placed immediately adjacent to the Off position 305 because the AED mode is the most time-critical mode of operation. The first subposition 340 for manual defibrillation is located immediately adjacent position to the Off position 305 on the opposite side from the AED position 320, because the manual mode is the most time-critical mode of operation after the AED mode. The Monitor position 330 is located away from the Off position 305, such that a user must switch through either a manual mode energy or an AED mode position from the Off position 305 in order to arrive at Monitor mode. In this embodiment, a time-critical mode must be selected first regardless of whether the rotation of the selector switch 300 is constrained in one direction or not.

Each of the embodiments described in FIGURES 2 and 3 solves the problem as identified by the inventors. In the event of a time-critical cardiac emergency, such as a sudden cardiac arrest in which rapid defibrillation is critical to the patient's survival, a defibrillator mode will be activated immediately upon rotating the switch one position from "Off." The few seconds time-saving of such a feature could mean the difference between patient survival and death. In addition, no opportunity arises to mistakenly switch the device into a non-therapeutic mode of operation with the attendant risk of delaying electrotherapy even further.

Other variations within the scope of the aforedescribed invention will readily occur to those skilled in the art. For instance, the particular identifying markings of the operating modes or the functionality of the underlying modes may vary within the scope of the claimed invention. Also, the scope of the invention may encompass digital displays, such that in selecting a particular operating mode, the most time-critical mode (e.g. AED mode) appears first in a sequential displayed list.

## Claims

1. A medical device for treating time-critical cardiac arrhythmias, comprising:
a rotatable mode selector switch (200, 300) having at least three positions including:
an off position (205) configured to inactivate the device from electrotherapy and monitoring;
a first position arranged to be immediately adjacent to the off position and configured to select a first electrotherapy mode of operation; and
a monitoring position (230) arranged to be not immediately adjacent to the off position and configured to select a cardiac monitoring mode of operation in which electrotherapy is inactivated.

2. The medical device of Claim 1, wherein the first electrotherapy mode of operation is an AED mode of operation.

3. The medical device of Claim 2, wherein the rotatable mode selector switch (200, 300) further comprises:
a third position configured to select a manual defibrillation mode of operation, wherein the third position is arranged between the first position and the monitoring position.

4. The medical device of Claim 2, wherein the rotatable mode selector switch (200, 300) further comprises:
a third position configured to select a manual defibrillation mode of operation, wherein the third position is arranged between the off position and the monitoring position.

5. The medical device of Claim 4, wherein the rotatable mode selector switch (200, 300) further comprises:
a fourth position configured to select a synchronized cardio-version mode of operation, wherein the fourth position is arranged between the third position and the monitoring position.

6. The medical device of Claim 5, wherein the rotatable mode selector switch (200, 300) further comprises:
a fifth position configured to select a pacing mode of operation, wherein the fifth position is arranged between the third position and the monitoring position.

7. The medical device of Claim 6, wherein the fifth position is arranged between the fourth position and the monitoring position.

8. The medical device of Claim 4, wherein the third position configured to select a manual defibrillation mode of operation further comprises a plurality of sub-positions, each subposition for selecting a unique energy for manual defibrillation.

9. The medical device of Claim 3, wherein the off position is arranged between the first position and the third position.

## Patentansprüche

1. Medizinische Vorrichtung zur Behandlung zeitkritischer Herzrhythmusstörungen, umfassend:
einen drehbaren Moduswählschalter (200, 300), der mindestens drei Positionen aufweist mit:
einer OFF-Position (205), die so eingerichtet ist, dass sie die Vorrichtung für Elektrotherapie und Überwachung deaktiviert;
einer ersten Position, die unmittelbar in Angrenzung an die OFF-Position angeordnet und so eingerichtet ist, dass sie einen ersten Elektrotherapiebetriebsmodus auswählt; sowie
eine Überwachungsposition (230), die nicht unmittelbar in Angrenzung an die OFF-Position angeordnet und so eingerichtet ist, dass sie einen Herzüberwachungsbetriebsmodus auswählt, in dem Elektrotherapie deaktiviert wird.

2. Medizinische Vorrichtung nach Anspruch 1, wobei der erste Elektrotherapiebetriebsmodus ein AED-Betriebsmodus ist.

3. Medizinische Vorrichtung nach Anspruch 2, wobei der drehbare Moduswählschalter (200, 300) weiterhin umfasst:
eine dritte Position, die so eingerichtet ist, dass sie einen manuellen Defibrillationsbetriebsmodus auswählt, wobei die dritte Position zwischen der ersten Position und der Überwachungsposition angeordnet ist.

4. Medizinische Vorrichtung nach Anspruch 2, wobei der drehbare Moduswählschalter (200, 300) weiterhin umfasst:
eine dritte Position, die so eingerichtet ist, dass sie einen manuellen Defibrillationsbetriebsmodus auswählt, wobei die dritte Position zwischen der OFF-Position und der Überwachungsposition angeordnet ist.

5. Medizinische Vorrichtung nach Anspruch 4, wobei der drehbare Moduswählschalter (200, 300) weiterhin umfasst:
eine vierte Position, die so eingerichtet ist, dass sie einen synchronisierten Kardioversionsbetriebsmodus auswählt, wobei die vierte Position zwischen der dritten Position und der Überwachungsposition angeordnet ist.

6. Medizinische Vorrichtung nach Anspruch 5, wobei der drehbare Moduswählschalter (200, 300) weiterhin umfasst:
eine fünfte Position, die so eingerichtet ist, dass sie einen Pacing-Betriebsmodus auswählt, wobei die fünfte Position zwischen der dritten Position und der Überwachungsposition angeordnet ist.

7. Medizinische Vorrichtung nach Anspruch 6, wobei die fünfte Position zwischen der vierten Position und der Überwachungsposition angeordnet ist.

8. Medizinische Vorrichtung nach Anspruch 4, wobei die dritte Position, die so eingerichtet ist, dass sie einen manuellen Defibrillationsbetriebsmodus auswählt, weiterhin mehrere Subpositionen umfasst, wobei jede Subposition zum Auswählen einer eindeutigen Energie zur manuellen Defibrillation dient.

9. Medizinische Vorrichtung nach Anspruch 3, wobei die OFF-Position zwischen der ersten Position und der dritten Position angeordnet ist.

## Revendications

1. Dispositif médical pour traiter des arythmies cardiaques en urgence, comprenant :
un commutateur sélecteur de mode rotatif (200, 300) possédant au moins trois positions incluant :
une position d'arrêt (205) configurée pour inactiver l'électrothérapie et la surveillance du dispositif ;
une première position agencée pour être immédiatement adjacente à la position d'arrêt et configurée pour sélectionner un premier mode de fonctionnement à électrothérapie ; et
une position de surveillance (230) agencée pour ne pas être immédiatement adjacente à la position d'arrêt et configurée pour sélectionner un mode de fonctionnement à surveillance cardiaque dans lequel l'électrothérapie est inactivée.

2. Dispositif médical selon la revendication 1, dans lequel le premier mode de fonctionnement à électrothérapie est un mode de fonctionnement AED.

3. Dispositif médical selon la revendication 2, dans lequel le commutateur sélecteur de mode rotatif (200, 300) comprend en outre :
une troisième position configurée pour sélectionner un mode de fonctionnement à défibrillation manuelle, dans lequel la troisième position est agencée entre la première position et la position de surveillance.

4. Dispositif médical selon la revendication 2, dans lequel le commutateur sélecteur de mode rotatif (200, 300) comprend en outre :
une troisième position configurée pour sélectionner un mode de fonctionnement à défibrillation manuelle, dans lequel la troisième position est agencée entre la position d'arrêt et la position de surveillance.

5. Dispositif médical selon la revendication 4, dans lequel le commutateur sélecteur de mode rotatif (200, 300) comprend en outre :
une quatrième position configurée pour sélectionner un mode de fonctionnement à cardio-version synchronisée, dans lequel la quatrième position est agencée entre la troisième position et la position de surveillance.

6. Dispositif médical selon la revendication 5, dans lequel le commutateur sélecteur de mode rotatif (200, 300) comprend en outre :
une cinquième position configurée pour sélectionner un mode de fonctionnement à stimulation, dans lequel la cinquième position est agencée entre la troisième position et la position de surveillance.

7. Dispositif médical selon la revendication 6, dans lequel la cinquième position est agencée entre la quatrième position et la position de surveillance.

8. Dispositif médical selon la revendication 4, dans lequel la troisième position configurée pour sélectionner un mode de fonctionnement à défibrillation manuelle comprend en outre une pluralité de sous-positions, chaque sous-position étant pour sélectionner une énergie unique pour la défibrillation manuelle.

9. Dispositif médical selon la revendication 3, dans lequel la position d'arrêt est agencée entre la première position et la troisième position.
